# EUROPEAN PATENT APPLICATION

(11) **EP 2 550 917 A1**
(43) Date of publication of application: **30.01.2013**
(21) Application number: 12164289.6
(22) Date of filing: 16.04.2012
(51) Int. Cl.: A61B 5/145, G01N 27/327

(54) **Multifunctional detector providing automatic identification of detection modes and test specimen used thereof**

(30) Priority: 27.07.2011 TW 100213808
(71) Applicant: Bioptik Technology, Inc., 350 Zhunan Township, Miaoli County (TW)
(72) Inventor: Hsu, Hsiang-Wei, 350 Zhunan Township, Miaoli County (TW); Wu, Kun-Lieh, 350 Zhunan Township, Miaoli County (TW); Yang, Chin-Chang, 350 Zhunan Township, Miaoli County (TW)
(74) Representative: 2K Patentanwälte Blasberg Kewitz & Reichel

(57) **Abstract**

A multifunctional detector includes a body (20), a test specimen insertion slot (30), a mode selection unit (40) and a processor (50) to test a test specimen (10A,10B,10C,10D) which includes a working electrode (11), a reference electrode (12) and at least one identification pin (13). The working electrode, reference electrode and identification pin are located at one end of the test specimen in a juxtaposed manner. The identification pin includes multiple sets electrically connecting with the working electrode to form multiple detection modes to test different physiological signals. After the test specimen is inserted into the test specimen insertion slot, the processor reads and gets the potential of the working electrode and identification pin to identify electric connection state between them and control the mode selection unit to select a corresponding detection mode to perform detection through the test specimen. Thus the detection modes of the test specimen can be indentified and selected automatically to improve usability, and also prevent erroneous selection of the detection modes to result in false detection of physiological signals to harm user's health.

## Description

### FIELD OF THE INVENTION

The present invention relates to a physiological signal detector and particularly to a multifunctional detector capable of providing automatic identification of detection modes and a test specimen used thereof.

### BACKGROUND OF THE INVENTION

For debilitated people of middle and senior age and bed-ridden patients, ailments such as high blood pressure, diabetes, gout, cardiovascular obstruction, anemia and the like are most likely to arise. Most middle and senior age people have potential factors to induce the aforesaid ailments. In order to discover the disease symptoms at the earliest time, it is generally encouraged for the middle and senior age people and patients to do self-monitoring of the physiological information such as blood pressure, blood sugar, cholesterol, uric acid and hemoglobin at home. In the event that the physiological information is abnormal, suitable actions can be taken timely to maintain the physical health of the people and patients.

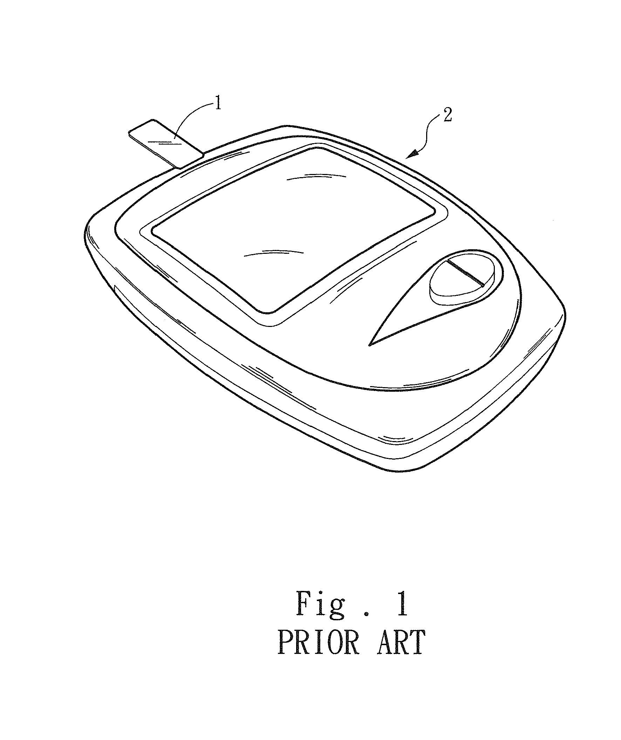

Please refer to FIG. 1 for a conventional test specimen 1 and reader 2 now available on the market. The test specimen 1, according to its design, can be used to detect various types of physiological information. After a user's specimen is dispensed thereon, such as blood, uric acid or the like, it can be inserted into the reader 2 to be read and interpreted, and the related physiological information such as blood sugar, uric acid, hemoglobin, triglyceride or the like can be obtained. To make correct detection and achieve higher accuracy of the detection result, a correct corresponding detection mode must be selected according to the intended physiological information to be detected via the test specimen 1 to measure and obtain accurate physiological information.

However, for middle and senior age people, it is difficult to select correct detection modes for different test specimens 1, especially when many different types of physiological information have to be detected, it is easy to make erroneous selection of the detection mode and result in incorrect detected physiological information. This could cause overlooking of the alarm messages to make false treatment and put user's health in danger.

### SUMMARY OF THE INVENTION

Therefore, the primary object of the present invention is to provide a multifunctional detector capable of providing automatic identification of detection modes and a test specimen used thereof. The multifunctional detector can receive insertion of the test specimen and automatically select a correct detection mode.

In order to achieve the foregoing object, the multifunctional detector of the invention is incorporated with a test specimen when in use. The test specimen includes a working electrode, a reference electrode and at least one identification pin. The working electrode, reference electrode and identification pin are located at one end of the test specimen in a juxtaposed manner. The identification pin has multiple sets electrically connecting with the working electrode to form multiple detection modes.

The multifunctional detector includes a body, a test specimen insertion slot, a mode selection unit and a processor. The test specimen insertion slot is located on the body to receive insertion of the test specimen. The mode selection unit is located in the body to be used for mode selection. The processor is located in the body and electrically connected to the mode selection unit and test specimen insertion slot to read and get potential of the working electrode, reference electrode and identification pin to identify electric connection state between the identification pin and working electrode and also control the mode selection unit to select a corresponding detection mode to perform detection through the test specimen.

By means of the structure set forth above, after the test specimen is inserted into the test specimen insertion slot of the multifunctional detector, through the potential identification of the identification pin, the detection mode of the test specimen can be automatically identified and selected, thus usability improves, and erroneous selection of the detection mode can be avoided that would result in false detection of physiological signals to harm user's health.

The foregoing, as well as additional objects, features and advantages of the invention will be more readily apparent from the following detailed description, which proceeds with reference to the accompanying embodiments and drawings. The embodiments serve merely for illustrative purpose and are not the limitations of the invention.

### BRIEF DESCRIPTION OF THE DRAWINGS

FIG. 1 is a schematic view of a conventional reader.
FIG. 2 is a perspective view of the invention.
FIG. 3 is a fragmentary exploded view of the invention.
FIG. 4 is a schematic view of the test specimen of the invention in electric connection state-1.
FIG. 5 is a schematic view of the test specimen of the invention in electric connection state-2.
FIG. 6 is a schematic view of the test specimen of the invention in electric connection state-3.
FIG. 7 is a schematic view of the test specimen of the invention in electric connection state-4.

### DETAILED DESCRIPTION OF THE PREFERRED

### EMBODIMENTS

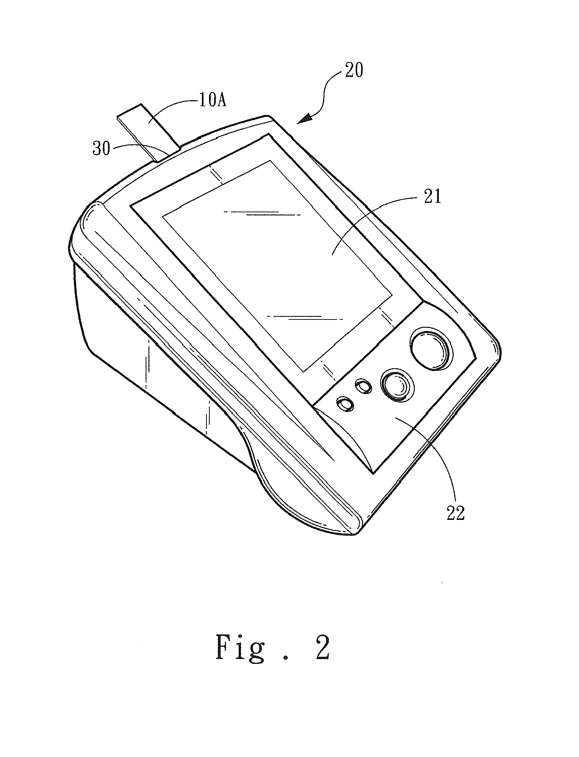

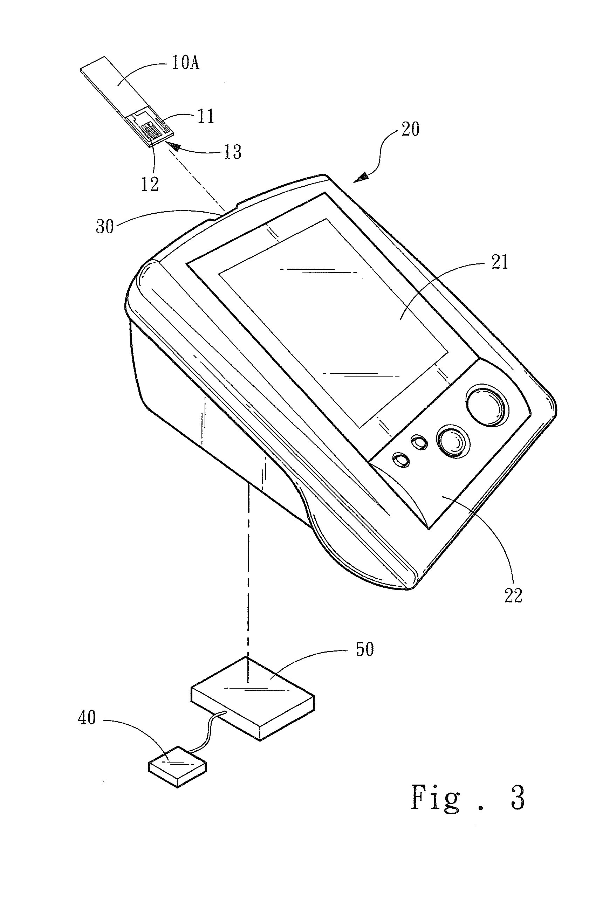

Please refer to FIGS. 2, 3 and 4, the present invention aims to provide a multifunctional detector capable of providing automatic identification of detection modes that is incorporated with a test specimen 10A when in use. The test specimen 10A includes a working electrode 11, a reference electrode 12 and at least one identification pin 13. The working electrode 11, reference electrode 12 and identification pin 13 are located at one end of the test specimen 10A in a juxtaposed manner. The identification pin 13 has multiple sets electrically connecting with the working electrode 11 to form multiple detection modes. The working electrode 1 also is connected to an electric power source and at a potential usually indicated by "1". The reference electrode 12 is grounded at a potential indicated by "0".

The multifunctional detector includes a body 20, a test specimen insertion slot 30, a mode selection unit 40 and a processor 50. The test specimen insertion slot 30 is located on the body 20 to receive insertion of the test specimen 10A. The mode selection unit 40 is located in the body 20 for selection of the teat mode. The processor 50 is located in the body 20 to form electric connection with the mode selection unit 40 and test specimen insertion slot 30 to read and get the potentials of the working electrode 11, reference electrode 12 and identification pin 13 to identify electric connection state between the identification pin 13 and working electrode 11 and also control the mode selection unit 40 to select a corresponding detection mode to perform detection through the test specimen 10A. The body 20 further can include a display device 21 to display information and a control key set 22 to provide input function to facilitate operation and control of the multifunctional detector.

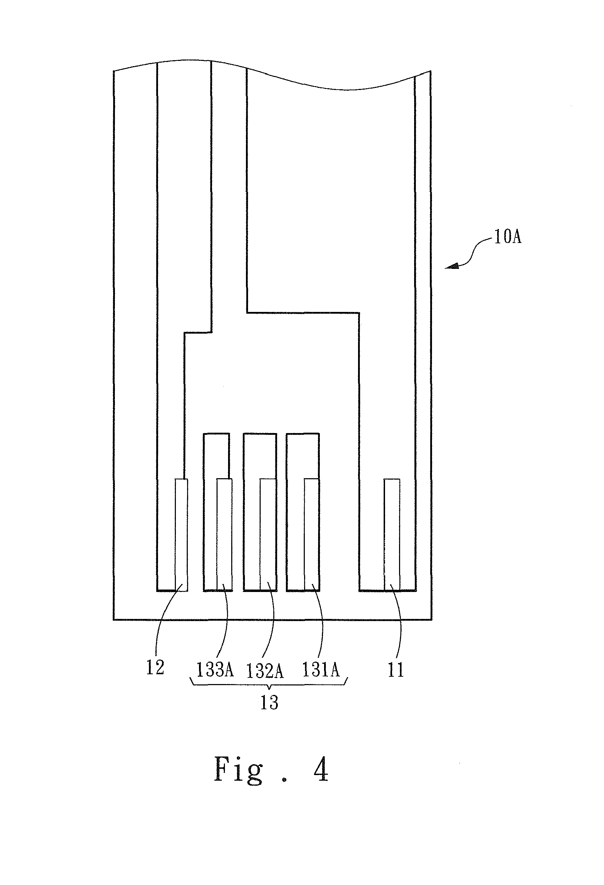

Please refer to FIG. 4, the identification pin 13 has multiple sets electrically connecting with the working electrode 11. In this embodiment, the identification pin 13 includes three sets to serve as an example that can be defined as a first identification pin 131A, a second identification pin 132A and a third identification pin 133A to form eight different electric connection states for eight detection modes to measure physiological information such as blood sugar, uric acid, cholesterol, triglyceride and the like. Some of the details are elaborated below:

In the event that the first identification pin 131 A, second identification pin 132A and third identification pin 133A do not form electric connection with the working electrode 11, the test specimen 10A is used for measuring blood sugar. When the test specimen 10A is inserted into the test specimen insertion slot 30, the potential of the first identification pin 131 A, second identification pin 132A and third identification pin 133A is "0" due to no electric connection with the working electrode 11.

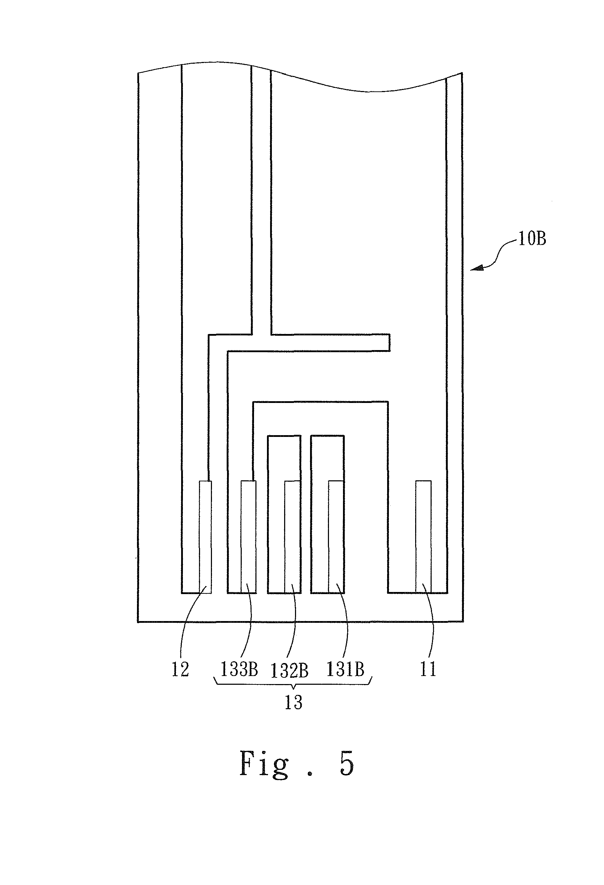

Please refer to FIG. 5 for another electric connection state between the identification pin 13 and working electrode 11, the test specimen 10B based on this electric connection state is used for measuring uric acid. The first identification pin 131B and second identification pin 132B do not form electric connection with the working electrode 11, while the third identification pin 133B forms electric connection with the working electrode 11, hence the potential of the first and second identification pins 131B and 132B is "0", while the potential of the third identification pin 133B is "1".

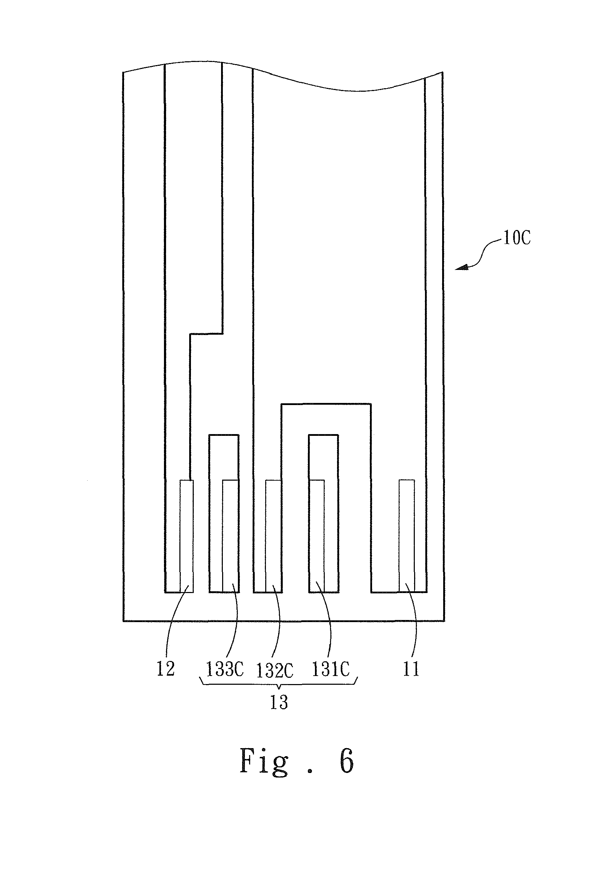

Please refer to FIG. 6 for yet another electric connection state between the identification pin 13 and working electrode 11, the test specimen 10C based on this electric connection state is used for measuring cholesterol. The first identification pin 131C and third identification pin 133C do not form electric connection with the working electrode 11, while the second identification pin 132C forms electric connection with the working electrode 11, hence the potential of the first and third identification pins 131C and 133C is "0", while the potential of the second identification pin 132C is "1".

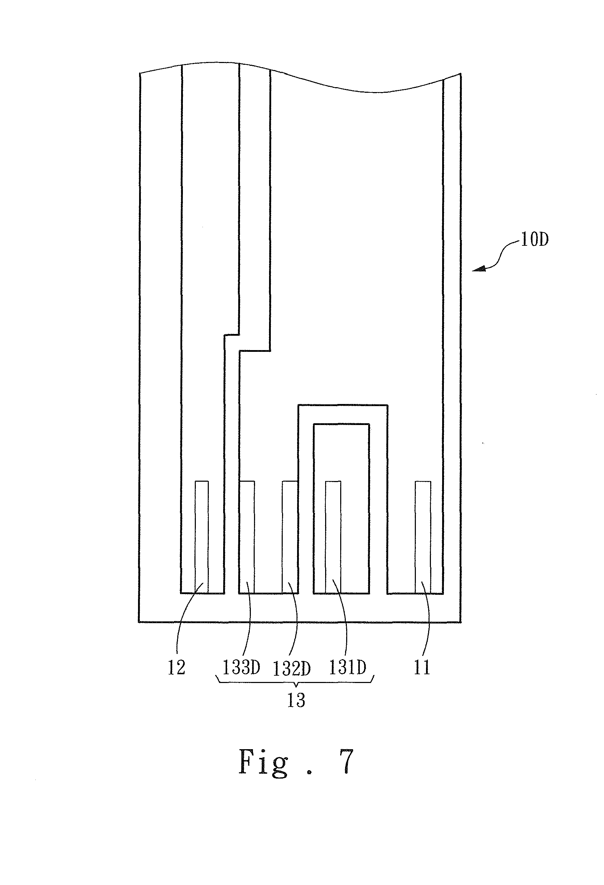

Please refer to FIG. 7 for still another electric connection state between the identification pin 13 and working electrode 11, the test specimen 10D based on this electric connection state is used for measuring triglyceride. The first identification pin 131D does not form electric connection with the working electrode 11, while the second identification pin 132D and third identification pin 133D form electric connection with the working electrode 11, hence the potential of the first identification pin 131D is "0", while the potential of the second identification pin 132D and third identification pin 133D is "1".

As a conclusion, when the test specimen 10A, 10B, 10C or 10D is inserted into the test specimen insertion slot 30, the processor 50 reads the potential of the working electrode 11 and the identification pin 13 to identify electric connection state between the identification pin 13 and working electrode 11, therefore can automatically identify the detection mode of the test specimen 10A, 10B, 10C or 10D to make automatic selection of measuring blood sugar, uric acid, cholesterol and triglyceride. As a result, usability improves, and erroneous selection of the detection mode can be avoided that would result in false detection of physiological signals to harm user's health.

## Claims

1. A multifunctional detector providing automatic identification of detection modes, incorporating with a test specimen (10A, 10B, 10C, 10D) which includes a working electrode (11), a reference electrode (12) and at least one identification pin (13) that are located at one end of the test specimen (10A, 10B, 10C, 10D) in a juxtaposed manner, the identification pin (13) including multiple sets electrically connecting with the working electrode (11) to form a plurality of detection modes, the multifunctional detector **characterized by**:
a body (20);
a test specimen insertion slot (30) located on the body (20) to receive insertion of the test specimen (10A, 10B, 10C, 10D);
a mode selection unit (40) located in the body (20) to provide selection of the plurality of detection modes; and
a processor (50) which is located in the body (20) and electrically connected to the mode selection unit (40) and the test specimen insertion slot (30), and reads potential of the working electrode (11) and the identification pin (13) to identify electric connection state between them and control the mode selection unit (40) to select one corresponding detection mode to perform detection through the test specimen (10A, 10B, 10C, 10D).

2. The multifunctional detector of claim 1, wherein the identification pin (13) of the test specimen (10A, 10B, 10C, 10D) includes three sets defined as a first identification pin (131A, 131B, 131C, 131D), a second identification pin (132A, 132B, 132C, 132D) and a third identification pin (133A, 133B, 133C, 133D).

3. The multifunctional detector of claim 1 or 2, wherein the body (20) includes a display device (21) and a control key set (22).

4. A test specimen **characterized by**:
a working electrode (11);
a reference electrode (12); and
at least one identification pin (13); the working electrode (11), the reference electrode (12) and the identification pin (13) being located at one end of the test specimen (10A, 10B, 10C, 10D) in a juxtaposed manner, the identification pin (13) including multiple sets electrically connecting with the working electrode (11) to form a plurality of detection modes.

5. The test specimen of claim 4, wherein the identification pin (13) of the test specimen (10A, 10B, 10C, 10D) includes three sets defined as a first identification pin (131A, 131B, 131C, 131D), a second identification pin (132A, 132B, 132C, 132D) and a third identification pin (133A, 133B, 133C, 133D).

6. The test specimen of claim 5, wherein the first identification pin (131A), the second identification pin (132A) and the third identification pin (133A) do not form electric connection with the working electrode (11).

7. The test specimen of claim 5 or 6, wherein the first identification pin (131B) and the second identification pin (132B) do not form electric connection with the working electrode (11), and the third identification pin (133B) forms electric connection with the working electrode (11).

8. The test specimen of any of the claims 5 to 7, wherein the first identification pin (131C) and the third identification pin (133C) do not form electric connection with the working electrode (11), and the second identification pin (132C) forms electric connection with the working electrode (11).

9. The test specimen of any of the claims 5 to 8, wherein the first identification pin (131D) does not form electric connection with the working electrode (11), and the second identification pin (132D) and the third identification pin (133D) form electric connection with the working electrode (11).
